**Europäisches Patentamt**

**European Patent Office**

**Office européen des brevets**

(11) Publication number: **0 190 856 B1**

# EUROPEAN PATENT SPECIFICATION

(45) Date of publication of patent specification:
**06.03.91 Bulletin 91/10**

(51) Int. Cl.⁵: **A61M 16/00, A61M 16/20**

(21) Application number: **86300521.1**

(22) Date of filing: **27.01.86**

(54) **Breathing apparatus.**

(30) Priority: **02.02.85 GB 8502681**

(43) Date of publication of application:
**13.08.86 Bulletin 86/33**

(45) Publication of the grant of the patent:
**06.03.91 Bulletin 91/10**

(84) Designated Contracting States:
**AT BE CH DE FR IT LI LU NL SE**

(56) References cited:
**US-A- 3 522 816**
**US-A- 4 202 330**

(72) Inventor: **Dearman, Peter Thomas**
**52 Thornbera Road**
**Bishop's Stortford Hertfordshire (GB)**
Inventor: **Hess, Christopher**
**52 Beeches Close**
**Saffron Walden Essex CB11 4BT (GB)**
Inventor: **Hess, Richard**
**Thyme House Walden Road Little Chesterford**
**Saffron Walden Essex CB2 1UD (GB)**
Inventor: **Kirk, Christine Ann**
**17 Larkswood**
**Harlow Essex CM17 9EE (GB)**
Inventor: **Wardell, Peter**
**36 De Vigier Avenue**
**Saffron Walden Essex CB10 2BN (GB)**

(74) Representative: **Gold, Tibor Zoltán et al**
**T.Z.GOLD & COMPANY 9, Staple Inn**
**London WC1V 7QH (GB)**

(73) Proprietor: **NEOTRONICS MEDICAL LIMITED**
**Parsonage Road Takeley**
**Bishop's Stortford Hertfordshire CM22 6PU**
**(GB)**

## Description

### Field of invention.

This invention relates to breathing apparatus for supplying pulses of breathable gas to a patient. Such apparatus is used for emergency resuscitation and also as a lung ventilator for persons under anaesthesia.

### Background to the invention.

Breathing apparatus of this type is known in a number of different forms. It is a characteristic of the apparatus that the gas feed to the patient is switched on and off at appropriate intervals, and it is known to use the breathable gas to control the valve movement. However, existing apparatus generally requires a considerable number of different components to provide the necessary regulatory function, and consumes an undesirably large proportion of the breathable gas for the purposes of driving the apparatus (see for example US-A-3522 816). The present invention aims to provide a solution to this problem.

### Summary of the invention.

According to the present invention, there is provided breathing apparatus for supplying pulses of breathable gas to a patient, the apparatus having an inlet, an outlet, a feed passage between the inlet and the outlet, and an ON/OFF valve in the feed passage, the valve including a piston working in a cylinder which is biased in one direction by a spring and is biased in an opposite direction by pressure in a pressure reservoir, wherein the apparatus includes a throttle downstream of the valve and the reservoir communicates with the passage between the throttle and the valve and includes a bleed valve to allow the reservoir pressure to decay.

The valve is preferably biased by the spring into the "passage open" position.

The throttle and/or the bleed valve are preferably adjustable to alter the respective through flow rates.

A flow restrictor may be placed between the feed passage and the reservoir, and this flow restrictor may also be adjustable. A one-way valve can be included at the entry to the reservoir.

The valve may comprise a hollow, tubular body which moves in a valve housing. Thus, in one arrangement, the inlet enters the valve housing between two spaced O-rings which seal against the tubular valve member, and the tubular valve member has holes through its tubular walls which communicate with the inlet when the holes are between the O-rings and which are shut off from the inlet when the holes are positioned outside the space defined by the O-rings.

The tubular member may form a piston rod attached to the piston which is biased in one direction by a spring and in an opposite direction by gas pressure.

A pressure reducing valve can be entered upstream of the inlet.

A preferred arrangement includes a by-pass passage for the valve, said passage including a spring loaded valve biased to close said by-pass passage except when the reservoir pressure lies below the normal residual pressure to which the pressure in the reservoir falls during ON/OFF cycling.

Additionally, the apparatus may include a spring loaded vent valve for the reservoir, openable to vent the reservoir and initiate an on-phase of the gas supply if the outlet pressure falls below a given threshold during an off-phase.

There may also be provided a by-pass line connecting from the inlet upstream of the valve to the outlet and an ON/OFF switch in said by-pass line which, when switched to the on-position, provides a continuous supply of gas to the outlet via the by-pass line prior to commencement of ON/OFF cycling.

### Brief description of the drawing.

The invention will now be further described, by way of example, with reference to the accompanying drawing in which :

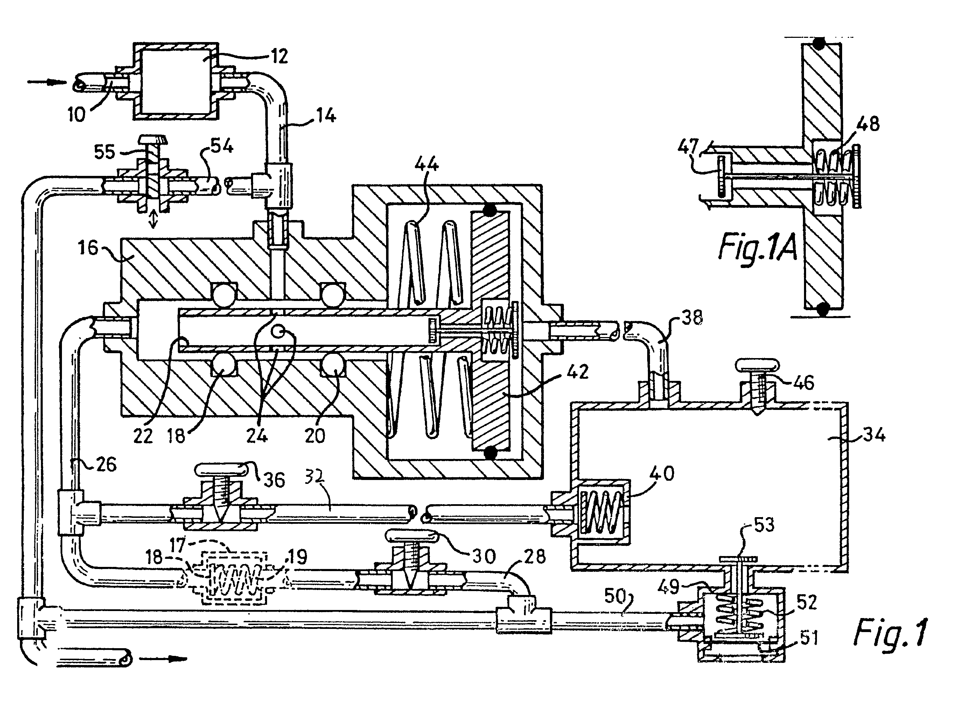

Figure 1 is a schematic view of breathing apparatus in accordance with the invention ; and Figure 2 shows a detail in enlargement.

### Description of the preferred embodiment.

Breathable gas (either air or oxygen) is introduced into a pipe 10 and passes through a pressure reducing valve 12 to an inlet 14 to the apparatus. The inlet 14 extends into a valve housing 16, into a space bounded by O-rings 18 and 20. A tubular valve member 22 slides through the O-rings such that an inlet chamber is formed between the O-rings around the tubular valve member. The valve member, which is hollow, has openings 24 through its wall so that, when the valve member is in the position shown in the drawing gas can flow through the openings through the tubular member 22, out through the open end of the tubular member and into a feed passage 26. The gas then leaves the apparatus through an outlet 28 which will be connected to a conventional patient valve.

A variable throttle 30 is positioned in the feed passage 26 and creates a back pressure causing gas to flow along a branch passage 32 into a reservoir 34. Another variable throttle 36 is provided in the passage 32, and a one-way valve 40 prevents reverse flow of gas through the passage 32.

Whilst the valve 16 is open, gas flows through the apparatus to the patient and also flows through the passage 32 into the reservoir 34, to build up the pressure in the reservoir. The reservoir is permanently connected through a control passage 38 to the reverse side of a piston 42 which controls movement of the valve member 22.

As the gas continues to flow, the pressure in the reservoir 34 builds up and thus the pressure acting against the piston 42 continually increases. At a certain pressure level, the pressure acting on the piston 42 is sufficient to overcome the biasing force of a coil spring 44, and the piston 40 together with the tubular member 22 is forced to the left. When this happens, the apertures 24 in the wall of the tubular member 22 pass beyond the O-ring 18 and are therefore closed off from the inlet 14. Gas therefore ceases to flow through the feed passage 26. In this condition there is no flow of gas through the outlet 28 to the patient, but the pressure in the reservoir 34 gradually decays through a vent 46 which is again adjustable. The pressure in the reservoir 34 thus drops gradually until the spring 44 is able to force the piston 42 to the right, overcoming the gas pressure in the reservoir. The valve then opens again and another pulse of gas is fed to the patient through the outlet 28, with the cycle repeating itself.

Because there is only a single pneumatically controlled valve, the apparatus is considerably simpler than previously known apparatus which achieves the same end. There are less components and less moving parts and the apparatus can be contained in a smaller package. A relatively high proportion of the gas introduced to the apparatus reaches the patient for the patients use with only a small proportion being wasted by venting through the vent 46.

The adjustable throttles 30 and 36 and the adjustable bleed valve 46 can be geared together so that the size and frequency of the gas pulses can he altered to suit the requirements of the patient.

Optionally, as indicated by the dashed lines, spring loaded throttle 17 generates additional back pressure in the system, enabling it to cycle at a lower input pressure. The gas in passage 26 has to attain a minimum threshold pressure to open the seal 18 against the closing action of the spring 19.

Most preferably, however, in the illustrated preferred arrangement, the throttle 17 is not employed, but on starting up (reservoir 34 empty), the initiation of cycling is advanced by means of a by-pass for the valve 16 to 24. The by-pass comprises a passage through the piston 42. This passage is normally closed at seal 47 due to the bias applied to the piston by the combined effect of spring 48 and the reservoir pressure. However on starting, when the reservoir pressure lies below even its minimum pressure level during cycling, the spring 44 is able to overcome the combined effect of the spring 48 and the reservoir pressure to allow opening of the valve by-pass passage at the seal 47, thereby to allow direct gas flow from the inlet to the reservoir in order rapidly to raise the reservoir pressure to the level at which cycling is initiated.

Additionally, at start up, the patient may be given a continuous supply of gas via by-pass line 54, which incorporates a manual ON/OFF switch 55. Line 54 is closed when the ON/OFF gas cycling process begins.

Finally, should the patient gasp for breath during an off-phase of the cycle, an on-stage may be immediately triggered through line 50 communicating the outlet 28 to the reservoir via valve 49. This valve includes a diaphragm 51 which is biased by a spring 52 to normally close the valve at seal 53. A negative pressure at the outlet (which is generated if a patient gasps for breath), causes the diaphragm 51 to lift, opening valve 49 at the seal 53 to instantly vent the reservoir and initiate an on-stage of the gas supply.

## Claims

1. Breathing apparatus for supplying pulses of breathable gas to a patient, comprising an inlet (14), an outlet (28), a feed passage (26) between the inlet and the outlet, and an ON/OFF valve (16) in the feed passage, wherein the valve is operable by a piston (42) working in a cylinder and which is biased in one direction by a spring (44) and is biased in the opposite direction by gas pressure in a pressure reservoir (34), a throttle (30) is provided in the feed passage (26) downstream of the valve, and the reservoir, which has a bleed valve (46) allowing the pressure in the reservoir to decay, communicates with the feed passage between the throttle and the valve.

2. Apparatus according to claim 1, characterised in that the valve piston is spring biased into a position in which the feed passage from inlet to outlet is normally open.

3. Apparatus according to claim 1 or claim 2 characterised in that the throttle and the bleed valve are adjustable for variation of the respective through flow rate.

4. Apparatus according to any of claims 1 to 3, characterised by a flow rate adjustable restrictor located between the feed passage and the reservoir, and a one-way valve at the entry to the reservoir downstream of said flow restrictor.

5. Apparatus according to any of claims 1 to 4, characterised in that the valve comprises a hollow tubular body which is movable in a valve housing to define an inlet chamber around the said valve member, in communication with the inlet, the tubular body having at least one aperture in its cylindical wall through which, in the ON position of the valve, the inlet chamber communicates with the interior of said valve member which thereby in turn communicates with the feed passage.

6. Apparatus according to claim 5, wherein the said tubular body forms a piston rod attached to the piston spring biased in one axial direction by the spring and in the opposite axial direction by the reservoir pressure.

7. Apparatus according to any of claims 1 to 6 characterised by a pressure reducing valve upstream of the valve.

8. Apparatus according to any of claims 1 to 7, characterised by a by-pass passage for the valve, said passage including a spring loaded valve biased to close said by-pass passage except when the reservoir pressure lies below the normal residual pressure to which the pressure in the reservoir falls during ON/OFF cycling.

9. Apparatus according to any of claims 1 to 8, characterised by a spring loaded vent valve for the reservoir, operable to vent the reservoir and initiate an ON-phase of the gas supply if the outlet pressure falls below a given threshold during an OFF-phase.

10. Apparatus according to any of claims 1 to 9, characterised by a by-pass line connecting from the inlet upstream of the valve to the outlet and an ON/OFF switch in said by-pass line which, when switched to the on-position, causes a continuous supply of gas to the outlet via the by-pass line prior to commencement of ON/OFF cycling.

## Ansprüche

1. Atmungsgerät zur Versorgung eines Patienten mit Stößen von atembarem Gas, das einen Einlaß (14), einen Auslaß (28), eine Speiseverbindung (26) zwischen dem Einlaß und dem Auslaß und ein EIN/AUS-Ventil in der Speiseverbindung enthält, wobei das Ventil durch einen Kolben (42) betätigbar ist, der in einem Zylinder arbeitet und der in einer Richtung durch eine Feder (44) vorgespannt ist und in der entgegengesetzten Richtung durch Gasdruck in einem Druckreservoir (34) vorgespannt ist, eine Drossel (30) in der Speiseverbindung (26) stromabwärts vom Ventil vorgesehen ist und das Reservoir, das ein Entlüftungsventil (46) hat, das dem Druck im Reservoir erlaubt abzunehmen, in Verbindung mit der Speiseverbindung zwischen der Drossel und dem Ventil steht.

2. Gerät gemäß Anspruch 1, dadurch gekennzeichnet, daß der Ventilkolben durch eine Feder in eine Position vorgespannt ist, in der die Speiseverbindung vom Einlaß zum Auslaß normalerweise offen ist.

3. Gerät gemäß Anspruch 1 oder 2, dadurch gekennzeichnet, daß die Drossel und das Entlüftungsventil einstellbar sind zur Variation der jeweiligen Durchflußrate.

4. Gerät nach einem der Ansprüche 1 bis 3, gekennzeichnet, durch einen einstellbaren Durchflußbegrenzer, der zwischen der Speiseverbindung und dem Reservoir angeordnet ist, und einem Einrichtungsventil an der Eintrittsstelle zu dem Reservoir stromabwärts von besagtem Durchflußbegrenzer.

5. Gerät nach einem der Ansprüche 1 bis 4, dadurch gekennzeichnet, daß das Ventil einen hohlen röhrenförmigen Körper enthält, der beweglich in einem Ventilgehäuse ist, um eine Einlaßkammer um das besagte Ventilglied zu definieren, das mit dem Einlaß in Verbindung steht, wobei der röhrenförmige Körper wenigstens eine Öffnung in seiner zylindrischen Wand hat, durch die in der EIN-Stellung des Ventils die Einlaßkammer mit dem Inneren des genannten Ventilgliedes in Verbindung steht, das dadurch wiederum in Verbindung mit der Speiseverbindung steht.

6. Gerät gemäß Anspruch 5, wobei der besagte röhrenförmige Körper eine Kolbenstange bildet, die an dem in einer Axialrichtung durch die Feder und in der entgegengesetzten Axialrichtung durch den Reservoirdruck federvorbelasteten Kolben befestigt ist.

7. Gerät nach einem der Ansprüche 1 bis 6, gekennzeichnet durch ein druckreduzierendes Ventil stromaufwärts von dem Ventil.

8. Gerät gemäß einem der Ansprüche 1 bis 7, gekennzeichnet durch eine Umgehungsleitung für das Ventil, wobei die besagte Leitung ein federbelastetes Ventil umfaßt, das vorgespannt ist, um die besagte Umgehungsleitung zu schließen, ausgenommen wenn der Reservoirdruck unterhalb des normalen Restdruckes liegt, auf den der Druck im Reservoir während eines EIN/AUS-Zykluses fällt.

9. Gerät nach einem der Ansprüche 1 bis 8, gekennzeichnet durch ein federbelastetes Entlüftungsventil für das Reservoir, das betätigbar ist, um das Reservoir zu entlüften und eine EIN-Phase der Gasversorgung zu initiieren, wenn während einer AUS-Phase der Ausgangsdruck unter eine vorgegebene Ansprechschwelle fällt.

10. Gerät nach einem der Ansprüche 1 bis 9, gekennzeichnet durch eine By-Pass-Leitungsverbindung vom Einlaß stromaufwärts von dem Ventil zum Auslaß und einem EIN/AUS-Schalter in besagter By-Pass-Leitung, der, wenn er in die EIN-Stellung geschaltet ist, eine ständige Versorgung des Auslasses mit Gas durch die By-Pass-Leitung bewirkt vor dem Beginn von EIN/AUS-Zyklen.

## Revendications

1. Appareil respiratoire pour la fourniture de pulsations d'un gaz respirable à un patient, comprenant une entrée (14), une sortie (28), un passage d'alimentation (26) entre l'entrée et la sortie, et un clapet MARCHE/ARRET (16) dans le passage d'alimentation, appareil dans lequel le clapet est actionnable par un piston (42) travaillant dans un cylindre et qui est soumis, dans une direction, à l'effet d'un ressort (44) et, dans l'autre direction, à une pression de gaz dans un réservoir de pression (34), un obturateur-régulateur (30) étant prévu dans le passage d'alimentation (26) en aval du clapet, et le réservoir qui a une soupape de fuite (46) permettant à la pression dans le réservoir de chuter, communiquant avec le passage d'alimentation entre l'obturateur-régulateur et le clapet.

2. Appareil selon la revendication 1, caractérisé en ce que le piston du clapet est soumis à l'effet d'un ressort qui tend à le mettre dans une position pour laquelle le passage d'alimentation allant de l'entrée à la sortie est normalement ouvert.

3. Appareil selon la revendication 1 ou 2, caractérisé en ce que l'obturateur-régulateur et la soupape de fuite sont réglables pour faire varier leur débit respectif.

4. Appareil selon l'une quelconque des revendications 1 à 3, caractérisé par un dispositif restricteur réglable du débit d'écoulement disposé entre le passage d'alimentation et le réservoir, et un clapet à sens unique à l'entrée du réservoir en aval dudit dispositif restricteur d'écoulement.

5. Appareil selon l'une quelconque des revendications 1 à 4, caractérisé en ce que le clapet comprend un corps tubulaire creux qui est mobile dans un logement du clapet pour définir une chambre d'entrée autour dudit élément de clapet, en communication avec l'entrée, le corps tubulaire ayant au moins une ouverture dans sa paroi cylindrique, ouverture au travers de laquelle, dans la position MARCHE du clapet, la chambre d'entrée communique avec l'intérieur dudit élément de clapet qui, à son tour, communique avec le passage d'alimentation.

6. Appareil selon la revendication 5, dans lequel ledit corps tubulaire forme une tige de piston fixée au piston soumis à l'effet d'un ressort dans une direction axiale et, dans la direction axiale opposée, à la pression du réservoir.

7. Appareil selon l'une quelconque des revendications 1 à 6, caractérisé par un clapet réducteur de pression en amont du clapet.

8. Appareil selon l'une quelconque des revendications 1 à 7, caractérisé par un passage de dérivation évitant le clapet, ledit passage comprenant un clapet soumis à l'effet d'un ressort tendant à fermer ledit passage de dérivation, excepté lorsque la pression du réservoir est inférieure à la pression résiduelle normale à laquelle tombe la pression dans le réservoir pendant le cycle MARCHE/ARRET.

9. Appareil selon l'une quelconque des revendications 1 à 8, caractérisé par une soupape d'évent soumise à l'effet d'un ressort équipant le réservoir, fonctionnant pour offrir une fuite au réservoir et commencer une phase de MARCHE de l'alimentation en gaz si la pression de sortie tombe au-dessous d'un seuil donné pendant une phase d'ARRET.

10. Appareil selon l'une quelconque des revendications 1 à 9, caractérisé par une ligne de dérivation allant de l'entrée en amont du clapet jusqu'à la sortie et un commutateur MARCHE/ARRET dans ladite ligne de dérivation qui, lorsqu'il est commuté sur la position MARCHE, provoque une alimentation continue en gaz vers la sortie via la ligne de dérivation avant le début du cycle MARCHE/ARRET.

Fig.1A

Fig.1